# EUROPEAN PATENT APPLICATION

(11) **EP 4 721 871 A1**
(43) Date of publication of application: **08.04.2026**
(21) Application number: 25198489.4
(22) Date of filing: 27.08.2025
(51) Int. Cl.: B05B 7/24, A61M 15/08, B05B 15/652, B05B 15/654, A61H 35/04, A61M 15/02, B05B 7/00

(54) **NASAL SHOWER DEVICE**

(30) Priority: 02.10.2024 IT 202400021819
(71) Applicant: 3A Health Care S.R.L., 25017 Lonato del Garda, BRESCIA (IT)
(72) Inventor: FRACCAROLI, Davide, 25017 Lonato del Garda, Brescia (IT); SOLDATI, Ruedi, 25017 Lonato del Garda, Brescia (IT); ABATE, Simone, 25017 Lonato del Garda, Brescia (IT)
(74) Representative: Chimini, Francesco

(57) **Abstract**

A nasal shower device (1; 100) comprises a base body (10) equipped with a dispensing nozzle (14) and an end element (16; 116) coupled to the base body (10). The end element includes a nosepiece (18; 118) suitable for receiving the medical substance in nebulized form dispensed by the dispensing nozzle (14). The end element (16; 116) is equipped with oscillation means (30; 130) designed to enable a user to position and maintain the nosepiece (18; 118) in an inclined operating position relative to the nozzle axis (Y).

## Description

The present invention relates to a nasal shower device.

Nasal shower devices are medical devices suitable for delivering a medical substance in nebulized form directly into the nostrils of a user. In the following description, medical substance also means simple saline water or other liquid that, when nebulized, helps dissolve mucus present in the upper respiratory tract.

An example of a nasal shower device is described in WO2013/072856, on behalf of the same Applicant. Such a device includes a chamber for collecting the medical substance and, superimposed and separate from it, a chamber for collecting the substance already used for washing and the mucus expelled from the nostrils. For the delivery of the substance in nebulized form, the device is equipped with a nozzle that can be connected to a source of compressed air, such as a compressor, and configured to suck the medical substance from the respective chamber by exploiting the Venturi effect. The nebulized medical substance exiting the nozzle is delivered through a nosepiece, shaped to be inserted into a user's nostril.

This type of nasal shower device has proven to be effective and functional. However, from an ergonomic point of view, a limitation was found to be related to the fact that, especially when the medical substance is running out, the device forces the user to assume a position that is not always comfortable in order to make use of all the substance in the collection chamber.

The object of the present invention is to propose a nasal shower device that can overcome such a limitation and thus be even more comfortable during use.

This object is achieved by a nasal shower device in accordance with claim 1. The dependent claims describe preferred or advantageous forms of embodiment of the device according to the invention.

However, the features and advantages of the nasal shower device according to the invention will be evident from the description below of its preferred embodiments, given by way of illustration and not limitation, with reference to the attached drawings, in which:
- Figure 1 is an elevation view of a nasal shower device according to the invention, in a first embodiment and in a use configuration with an inclined nosepiece;
- Figure 2 is a plan view from above of the nasal shower device of Figure 1;
- Figure 3 is an axial section of the nasal shower device, according to line A-A in Figure 2;
- Figure 4 is an axial section of the nasal shower device, according to the B-B line in Figure 2;
- figure 5 is an exploded view of the nasal shower device of figure 1;
- figure 6 is an elevation view of a nasal shower device according to the invention, in a second embodiment and in a use configuration with an inclined nosepiece;
- Figure 7 is a plan view from above of the nasal shower device of Figure 6;
- Figure 8 is an axial section of the nasal shower device, according to line A-A in Figure 6;
- Figure 9 is an axial section of the nasal shower device, according to line B-B in Figure 6; and
- figure 10 is an exploded view of the nasal shower device of figure 10.

In said drawings, 1; 100 has been used to indicate a nasal shower device according to the invention as a whole.

In the following description, all directional references (e.g., top, bottom, up, down, left, right, left, right, top, bottom, top, bottom, vertical, horizontal, clockwise, and counterclockwise) are used only for identification purposes to help the reader understand the described forms of realization and do not create any limitations, particularly regarding the location, orientation, or use of the described forms of realization.

Conjunction references (e.g., fixed, coupled, connected, and the like) should be interpreted broadly and can include intermediate elements between a connection of elements and relative movement between elements. Therefore, conjunction references do not necessarily imply that two elements are directly connected and in fixed relationship to each other.

In addition, elements common to the different embodiments will be given the same numerical references.

In a general embodiment, the nasal shower device 1; 100 includes a base body 10. The base body 10 extends mainly along a main axis X, which for simplicity of exposition can be assumed to be vertical when the base body is resting on a plane. In one embodiment, in fact, the base body 10 has a lower end 10' configured to act as a base resting on a plane.

The base body 10 defines a first chamber 12 to accommodate a medical substance to be nebulized.

The base body 10 is also provided with a dispensing nozzle 14 suitable for being fluidically connected to a source of compressed air, such as a compressor, to deliver the medical substance in nebulized form.

For example, dispensing nozzle 14 has a lower end portion 14' suitable for coupling to a hose connection to the compressed air source. Dispensing nozzle 14 terminates at the top with a dispensing hole 14".

The nasal shower device 1; 100 also includes an end element 16; 116 suitable for coupling to base body 10, specifically to an upper end portion 10" of base body 10.

The end element 16; 116 includes a nosepiece 18; 118 suitable for receiving the medical substance in nebulized form dispensed from the dispensing nozzle 14 and conveying it to a user's nostril through an outlet hole 20; 120 made in the distal end of the nosepiece 18; 118.

In other words, the nosepiece 18; 118 is a substantially tubular element having a lower, or proximal, end 22; 122 that is open to the dispensing hole 14" of the medical substance nebulized by the dispensing nozzle 14, and an upper, or distal, end 24; 124, in which the outlet hole 20; 122 is made, which, in use, is located within a user's nostril.

In accordance with one aspect of the invention, the end element 16; 116 is provided with oscillation means 30; 130 adapted to allow a user to carry and maintain the nosepiece 18; 118 in a position of use at an angle relative to the dispensing nozzle 14.

In other words, the nosepiece 18; 118 is jointed with respect to the basic body 10, so that the user can carry out the treatment in the position that is most comfortable for him, also in view of the amount of medical substance in the first chamber 12.

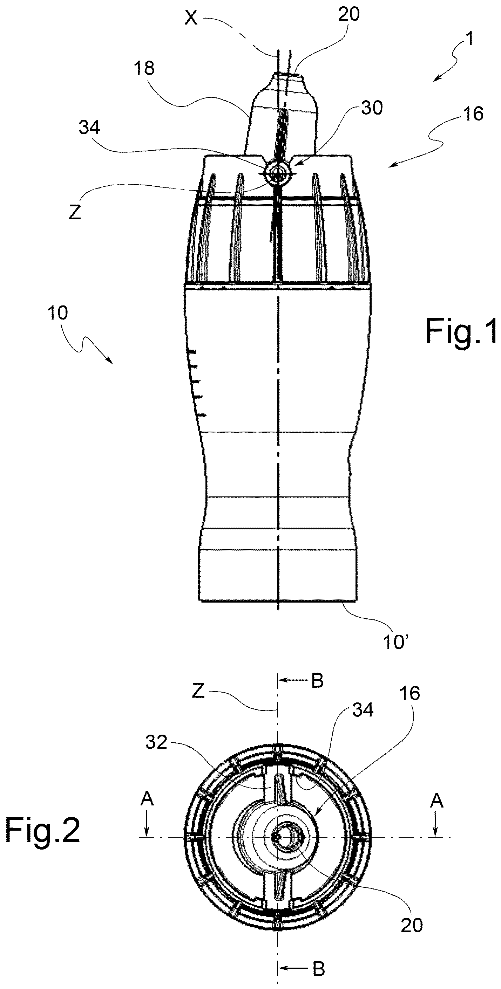

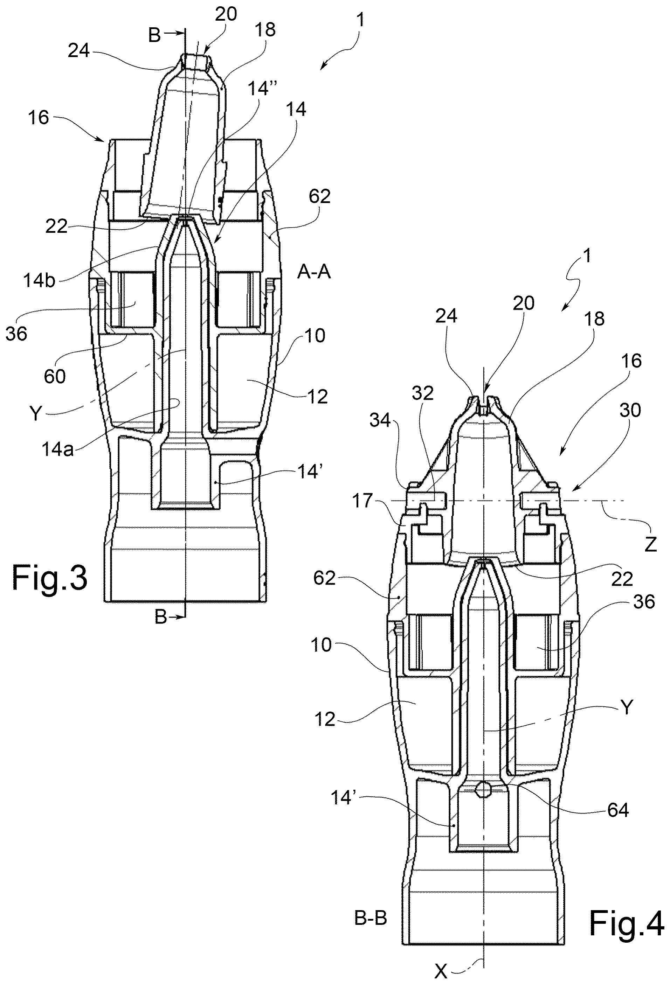

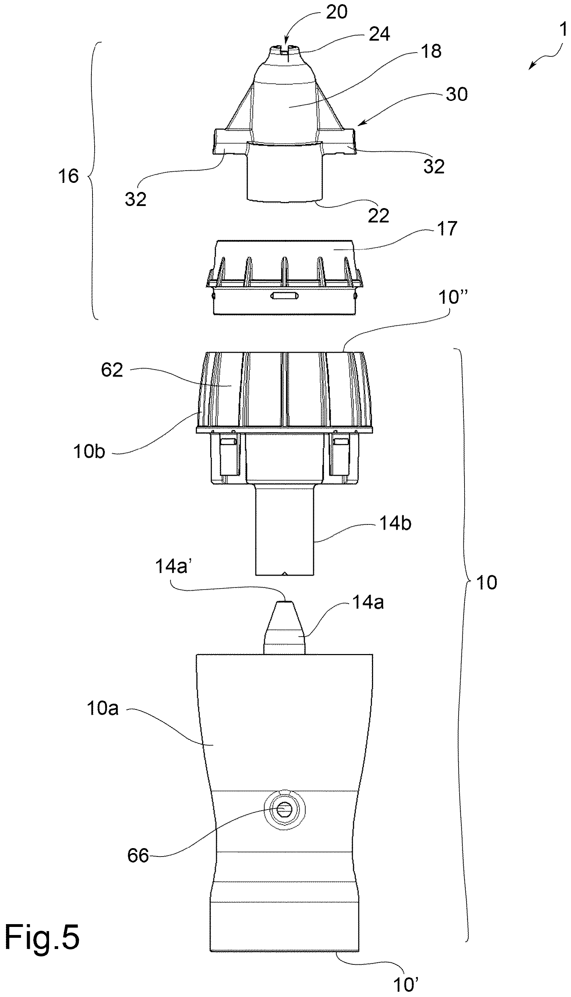

In a first embodiment illustrated in Figures 1-5, the end element 16 is suitable for oscillating around an axis of oscillation Z.

In more detail, defined by Y as the nozzle axis along which the dispensing nozzle 14 predominantly extends (and which is typically coincident with or parallel to the main X axis of the base body), the oscillation means 30 are suitable for allowing oscillation of the nosepiece 18 around an oscillation axis Z orthogonal to the Y nozzle axis.

In an embodiment, the end element 16 includes two diametrically opposed swing arms 32 extending from the nosepiece 18.

In an embodiment illustrated in Figures 1-5, the end element 16 includes a nosepiece support portion 17 to which the nosepiece 18 is hinged by means of the swing arms 32 and which is configured to couple, e.g., by snap or force coupling, to the distal, or upper, portion 10", of the base body 10.

For example, swing arms 32 are rotatably supported with calibrated interference by the nosepiece support portion 17.

In an embodiment, each swing arm 32 is at least partially accommodated with form fit coupling in a respective arm seat 34 made in the supporting nosepiece 17.

For example, arm seat 34 can be made as a split, or interruption, e.g., substantially circular in shape, of a side wall 17' of the nosepiece support portion 17.

In one embodiment, the nosepiece 18 with the swing arms could be supported directly by the upper end portion 10" of the base body 10. In other words, the nosepiece support portion 17 could be made integrally in one piece with the base body 10.

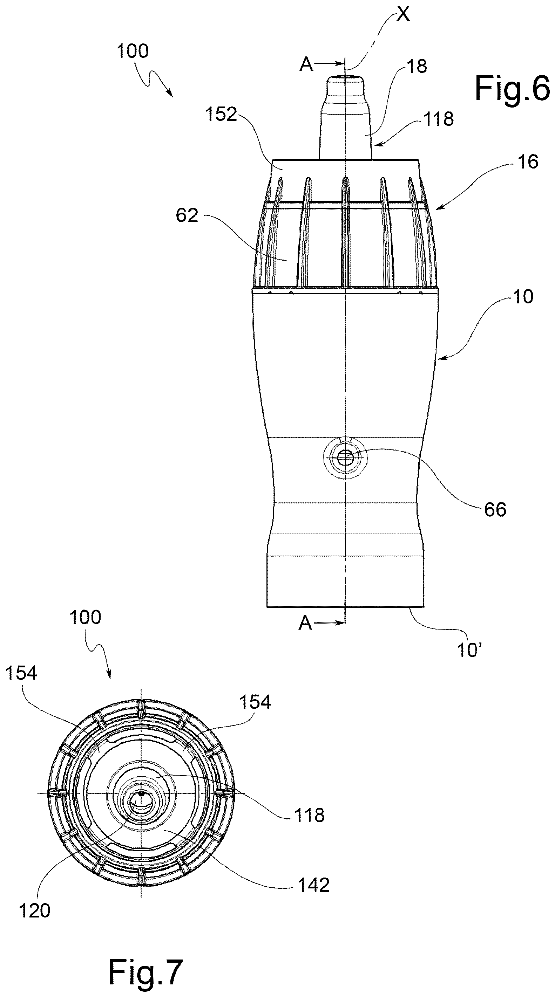

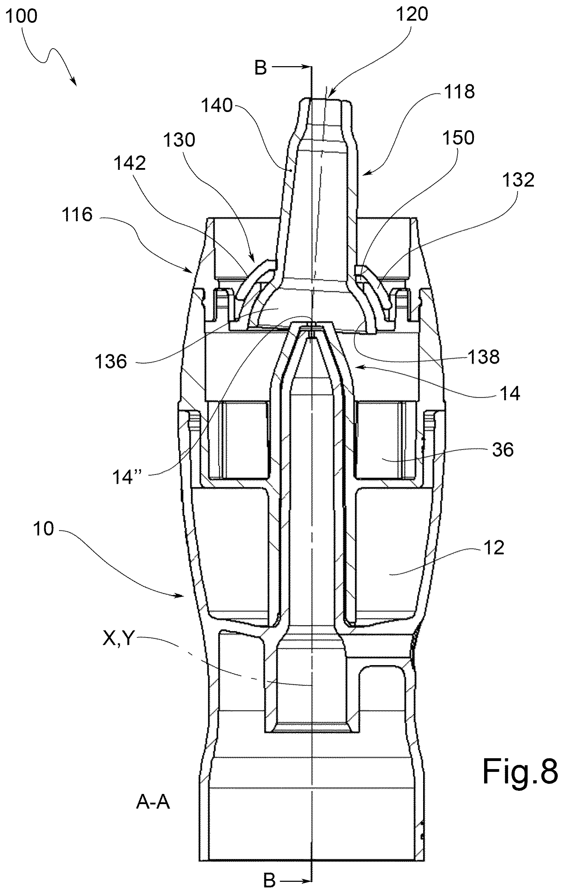

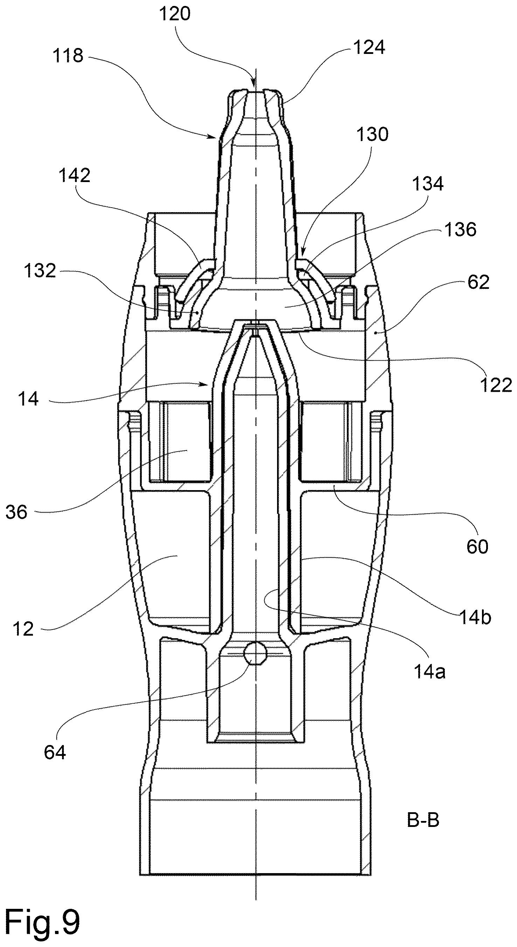

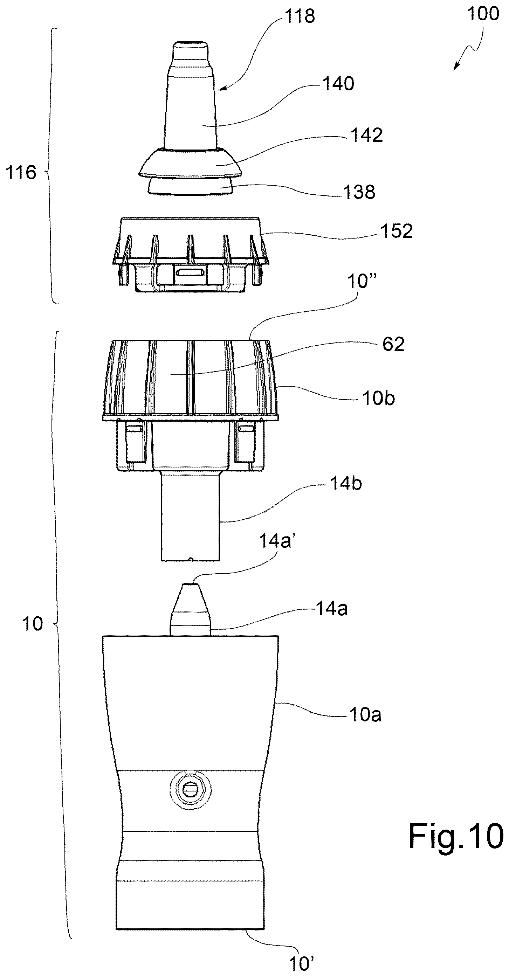

The nosepiece support portion 17 separated from the base body 10 can serve as an adapter element to allow the same base body 10 to receive different and interchangeable end elements, for example, with fixed nosepiece or with swiveling nosepiece as in the embodiment of Figures 6-10, described below.

In one embodiment illustrated in Figures 6-10, the oscillation means 130 are suitable to allow oscillation of the nosepiece 118 around infinite axes of oscillation orthogonal to the nozzle axis Y.

Specifically, the end element 116 includes a nosepiece support flange 132. The nosepiece 118 and the nosepiece support flange 132 are connected together to make a ball joint, i.e., a universal joint.

In an embodiment, the nosepiece support flange 132 is configured as an annular portion of spherical cap forming a flange central opening 134 and a flange concavity 136 facing the dispensing nozzle 14.

The nosepiece 118 has a spherically shaped nosepiece base 138 accommodated with shape coupling in the flange concavity 136 and a nosepiece head 140 that passes through the flange central opening 134 with the possibility of oscillation.

The nosepiece 118 is axially constrained to the nosepiece support flange 132 by means of a nosepiece ferrule 142 placed around and rigidly integral with the nosepiece head 140. The nosepiece ferrule 142 is configured to slide over the nosepiece support flange 132.

In other words, the nosepiece ferrule 142 also has a spherical cap shape with essentially the same radius of curvature as that of the nosepiece support flange 132.

Therefore, the nosepiece base 138 and nosepiece ferrule 142 jointly define a flange seat 150 slidingly engaged by the nosepiece support flange 132.

The nosepiece support flange 132, then, by engaging the respective flange seat 150, allows the nosepiece 118 to swing in any direction while axially constraining the nosepiece 118 to the end element 116.

In an embodiment, the nosepiece support flange 132 extends from a peripheral portion 152 of the end element 116. Such a peripheral portion 152, for example, is suitable for snap coupling to the distal end 10" of the base body 10.

In addition, between the nosepiece support flange 132 and the peripheral portion 152 of the end element 116, end element openings 154 are made to allow the passage of the flushing fluid already used in the underlying second chamber 36.

Note that, in an embodiment, the nosepiece lower end 18; 118, either in the version oscillating about an axis of oscillation or in the version with a ball joint, lies in a nosepiece end plane that, when the nosepiece 18; 118 is in the position of alignment with the nozzle axis Y, is substantially tangent to the dispensing hole 14" of the nozzle 14. This ensures that all of the nebulized substance exiting from that dispensing hole 14" is conveyed into the nosepiece 18; 118 even when the latter is in the tilted position.

In an embodiment, the distal portion 10" of the base body, i.e., its upper portion, is made by an annular wall that forms the upper part of a second chamber 36 suitable for collecting the already used washing fluid that escapes from the user's nostrils.

In an embodiment, the base body 10 includes a base body lower portion 10a and a base body upper portion 10b fit over the base body lower portion 10a. For example, the base body upper portion 10b is snap-coupled or with calibrated interference, to the base body lower portion body 10a.

As mentioned above, the base body lower portion 10a, moreover, is configured to serve as a base to allow the nasal shower device 1;100 to self-support itself by resting on a plane.

The dispensing nozzle 14 is made in such a way to suck the medical substance in the first chamber 12 by means of the Venturi effect. For this purpose, the dispensing nozzle 14 is formed by an inner nozzle element 14a and an outer nozzle element 14b, or nozzle cover, shod at least partially on the inner nozzle element 14a so as to form with the inner nozzle element 14a one or more suction channels of the medical substance. As is well known, the Venturi effect is exploited in these nasal shower devices by passing a flow of compressed air through the outlet hole 14a' of the inner nozzle element 14a. In this way, a vacuum is created between inner nozzle element 14a and outer nozzle element 14b, due to which the medical substance in the first chamber 12 is sucked towards the outlet hole 14' of the dispensing nozzle 14.

The base body lower portion 10a forms the first chamber 12, that is, the chamber for collecting the medical substance. In addition, the base body lower portion 10a forms the inner element of nozzle 14a, which is suitable to be fluidically connected to the compressed air source.

The base body upper portion 10b forms the second chamber 36, fluidically separated from the first chamber 12, to allow the collection of the medical substance already used for washing.

In addition, the base body upper portion 10b forms the nozzle outer element 14b.

In an embodiment, the base body upper portion 10b forms a separation septum 60 between second chamber 36 and first chamber 12. This separation septum 60 extends radially around the nozzle outer element 14b.

More specifically, the separation septum 60 extends from an intermediate portion of the nozzle outer element 14b. Thus, a lower portion of the nozzle outer element 14b extends inferiorly from the separation septum 60 into the first chamber 12, while an upper portion, terminating with the dispensing hole 14", extends into the second chamber 36.

Therefore, in an embodiment, the base body upper portion 10b forms the nozzle outer element 14b, separation septum 60, and, from the outer edge of separation septum 60, an annular wall 62 that laterally borders the second chamber 36.

In an embodiment, each of the base body lower portion 10a and base body upper portion 10b is integrally made in one piece.

Therefore, in an embodiment, the nasal shower device according to the invention is formed by only three parts: the base body lower portion 10a, base body upper portion 10b, and the end element 16; 116 (which can in turn be integrally made in one piece or in two separate parts).

The manufacturing process and assembly of the device are simplified compared to state-of-the-art devices.

In an embodiment, in addition, a trigger channel 64 is made into the base body 10 that fluidically intercepts the passage for compressed air from the dispensing nozzle 14 and externally flows to a trigger hole 66 made into an outer side wall of the base body 10 so that it can be easily occluded by the user by finger pressure.

To the embodiments of the nasal shower device according to the invention, a person skilled in the art, in order to meet contingent needs, may make modifications, adaptations and replacements of elements with functionally equivalent ones, without departing from the scope of the following claims. Each of the features described as belonging to a possible embodiment may be realized independently of the other described embodiments.

## Claims

1. Nasal shower device (1; 100), including:
- a base body (10) defining a first chamber (12) to accommodate a medical substance and fitted with a dispensing nozzle (14) suitable to be fluidically connected to a source of compressed air to deliver the medical substance in nebulized form, the dispensing nozzle (14) extending along a nozzle axis (Y);
- an end element (16; 116) coupled to the base body (10) and comprising a nosepiece (18; 118) suitable for receiving the medical substance in nebulized form dispensed from the dispensing nozzle (14) and conveying it to a user's nostril through an outlet hole (20; 120) made at the distal end of the nosepiece (18; 118),
wherein the end element (16; 116) is provided with oscillation means (30; 130) suitable for allowing a user to move and maintain the nosepiece (18; 118) in a position of use inclined with respect to the nozzle axis (Y),
the device being **characterized in that** the oscillation means (30) are suitable for allowing oscillation of the nosepiece (188) about infinite axes of oscillation orthogonal to the nozzle axis (Y).

2. A device according to claim 1, in which the end element (116) includes a nosepiece support flange (132), and in which the nosepiece (118) and the nosepiece support flange (132) are connected to each other so as to make a ball joint.

3. Device according to claim 2, in which:
- the nosepiece support flange (132) is configured as an annular portion of spherical cap forming a central flange opening (134) and a flange concavity (136) facing the dispensing nozzle (14);
- the nosepiece (118) has a spherically shaped nosepiece base (138) accommodated with shape coupling in the flange concavity (136) and a nosepiece head (140) that passes through the central flange opening (134) with the possibility of oscillation;
- the nosepiece (118) is axially constrained to the nosepiece support flange (132) by means of a nosepiece ferrule (142) placed around and rigidly integral with the nosepiece head (140), the nosepiece ferrule (142) being configured to slide over the nosepiece support flange (132) .

4. Device according to any of the preceding claims, wherein the base body (10) comprises a base body lower portion (10a) and a base body upper portion (10b) fitted on the base body lower portion, and wherein:
- the base body lower portion(10a) forms the first chamber (12) and an inner nozzle element (14a) suitable for being fluidically connected to the compressed air source;
- the base body upper portion(10b) forms a second chamber (36), fluidically separated from the first chamber so as to allow the collection of the medical substance already used for washing, and an outer nozzle element (14b) suitable for being shod at least partially on the inner nozzle element to cooperate with it in the delivery of the medical substance by Venturi effect.

5. Device according to claim 4, in which each of the base body lower portion and base body upper portion is integrally made in one piece.

6. Device according to claim 4 or 5, in which the base body upper portion(14b) forms a separation septum (60) between the second chamber (36) and the first chamber (12), the separation septum extending radially around the outer nozzle element (14b).

7. Device according to any one of the preceding claims, wherein a trigger channel (62) is formed in the base body (10) which fluidically intercepts the passage for compressed air from the dispensing nozzle and externally flows to a trigger hole (64) formed on an outer side wall of the base body (10) so as to be easily occluded by the user by finger pressure.
